# FASCICULE DE BREVET EUROPEEN

(11) **EP 1 121 100 B1**
(45) Date de publication et mention de la délivrance du brevet: **16.04.2003**
(21) Numéro de dépôt: 99947567.6
(22) Date de dépôt: 13.10.1999
(51) Int. Cl.: A61K 9/00, A61K 47/40, A61K 31/565, A61K 47/48

(54) **PROCEDE DE PREPARATION D'UNE SOLUTION POUR PULVERISATION NASALE CONTENANT UNE OU PLUSIEURS HORMONES SEXUELLES ET UNE CYCLODEXTRINE**
VERFAHREN ZUR HERSTELLUNG EINER ZUR NASALEN ZERSTÄUBUNG LÖSUNG, DIE EIN ODER MEHRERE SEXUALHORMONE ENTHÄLT
METHOD FOR PREPARING A SOLUTION FOR NASAL SPRAY CONTAINING SEX HORMONES AND A CYCLODEXTRIN

(30) Priorité: 14.10.1998 FR 9812836
(43) Date de publication de la demande: 08.08.2001
(73) Titulaire: LES LABORATOIRES SERVIER, 92200 Neuilly sur Seine (FR)
(72) Inventeur: FONKNECHTEN, Gilles, F-45000 Orléans (FR); WUTHRICH, Patrick, F-45000 Orléans (FR)
(86) Numéro de dépôt international: FR9902480
(87) Numéro de publication internationale: WO00021503

(56) Documents cités:
- EP-A- 0 349 091
- EP-A- 0 463 653
- WO-A-94/02518
- WO-A-94/22450
- DE-A- 4 207 922

## Description

La présente invention concerne un procédé de préparation d'une solution pour pulvérisation nasale contenant une ou plusieurs hormones sexuelles et une cyclodextrine.

L'administration par voie nasale d'hormones sexuelles présente de nombreux avantages par rapport aux autres voies d'administration classiquement utilisées.

Chez la femme, la carence oestrogénique est responsable à court terme des troubles climatériques chez 70 % des femmes, à plus long terme d'une ostéoporose chez 30 % d'entre elles et d'une augmentation du risque cardiovasculaire. Le traitement substitutif de la ménopause reste confronté à des difficultés de compliance qui n'ont qu'en partie été améliorées par les formes pharmaceutiques actuellement disponibles.

Les comprimés qui sont largement utilisés ont l'avantage de l'ancienneté et de la simplicité mais leurs dosages manquent de souplesse pour l'adaptation posologique. De plus, le phénomène de premier passage intestinal et hépatique est responsable de leurs inconvénients métaboliques. Les systèmes transdermiques permettent d'éviter ce phénomène de premier passage mais présentent des inconvénients importants comme la mauvaise tolérance locale, la variabilité des doses délivrées et l'adaptation difficile des doses. La voie nasale permet une administration de principe actif à visée systémique en évitant le phénomène de premier passage hépatique tout en permettant une adaptation aisée des doses administrées par simple modification du nombre de pulvérisations.

Le brevet EP 349 091 décrit une formulation destinée à l'administration nasale chez les femmes d'hormones sexuelles comme le 17-β-estradiol ou la progestérone et contenant une cyclodextrine.

Or, le développement d'une telle formulation implique que la préparation d'une solution pour pulvérisation nasale remplisse des contraintes industrielles telles qu'elle soient admissibles pour l'enregistrement d'un produit et permettent d'obtenir les meilleurs rendements, la fiabilité, la reproductibilité et la sécurité nécessaires à tout procédé industriel.

D'autre part, la solution nasale telle que décrite dans le brevet EP 349 091 contient, comme c'est souvent le cas, un agent conservateur comme le chlorure de benzalkonium. Or, lors d'une étude de l'efficacité antimicrobienne du chlorure de benzalkonium au sein d'une formulation nasale contenant du 17-β-estradiol et une cyclodextrine partiellement méthylée, il a été montré que l'efficacité antimicrobienne de ce conservateur n'était pas suffisante.

D'autre part, le chlorure de benzalkonium pouvant entraîner des réactions allergiques, il était nécessaire de préparer des solutions stériles sans conservateur en respectant les contraintes industrielles citées plus haut.

Enfin, les solutions pour l'administration nasale d'hormones sexuelles doivent être réalisées avec un solvant acceptable pharmacologiquement et n'entraînant pas de phénomènes d'irritation de la muqueuse nasale. Dans ce cas, une solution aqueuse au pH physiologique reste la meilleure des alternatives.

Or, les hormones sexuelles sont, la plupart d'entre elles, insolubles dans l'eau. L'utilisation d'une cyclodextrine comme décrit dans le brevet EP 349 091 permet de solubiliser en milieu aqueux ces composés lipophiles par formation d'un complexe d'inclusion.

Le problème à résoudre alors était le procédé de préparation industrielle de cette solution aqueuse, ce qui est l'objet de la présente invention. Plus spécifiquement, ce procédé concerne la préparation d'une solution aqueuse nasale contenant une ou plusieurs hormones sexuelles et une cyclodextrine.

Parmi les hormones sexuelles utilisables dans les compositions pharmaceutiques préparées selon le procédé de l'invention, on peut citer à titre non limitatif les oestrogènes stéroïdiens naturels comme l'estradiol, l'oestrone et leurs dérivés, les estrogènes stéroïdiens de synthèse comme l'éthinylestradiol, les progestatifs comme la progestérone, les pregnanes dérivés de la progestérone ou de la 17-α-OH-progestérone comme dydrogestérone, l'acétate de chlormadinone, la médrogestérone, l'acétate de médroxyprogestérone, les norpregnanes comme la démégestone, la promégestone, l'acétate de nomégestrol ou les dérivés de la 19-nortestostérone, comme la noréthistérone, le diacétate d'éthynodiol, le norgestrel, le lévonorgestrel, le désogestrel, le gestodène ou le norgestimate, et enfin, les antrogènes comme la testostérone et ses dérivés.

Plus spécifiquement, les compositions pharmaceutiques nasales préparées selon le procédé de l'invention sont celles qui contiennent une cyclodextrine partiellement méthylée.

Une composition préférée préparée selon le procédé de l'invention est une composition contenant de l'estradiol, un mélange estradiol/progestatif ou un androgène et une cyclodextrine méthylée partiellement et de manière randomisée.

Lorsque la composition contient un progestatif, le progestatif préféré est la noréthistérone (acétate).

Compte-tenu de l'insolubilité des hormones en milieu aqueux, le complexe d'inclusion hormone(s)/cyclodextrine peut être préparé classiquement par dissolution de la cyclodextrine et de (des) hormones(s) en milieu éthanol pur, ce qui permet la solubilisation des hormones et ainsi la formation du complexe d'inclusion. L'éthanol est alors évaporé sous vide vers 40°C et la solution aqueuse est obtenue par addition d'eau purifiée au complexe ainsi formé.

Ce procédé classique n'est pas envisageable, ni recommandé au niveau industriel. En effet, il nécessite l'évaporation à grande échelle d'éthanol absolu et sa récupération sous vide impose l'utilisation de matériel industriel antidéflagrant, ce qui, outre le danger que cela représente, augmente considérablement le coût industriel de fabrication de la solution.

D'autre part, l'utilisation d'un solvant comme l'éthanol impose le dosage de solvants résiduels, ce qui est bien entendu une contrainte supplémentaire dans la fabrication.

Ce type de procédé classique n'étant pas satisfaisant, le déposant a cherché à mettre au point un procédé de préparation de cette solution pour administration nasale n'utilisant plus de solvant comme l'éthanol, ce qui est l'objet de la présente invention.

Plus particulièrement, ce procédé est caractérisé en ce que la cyclodextrine est dissoute dans de l'eau purifiée à une concentration comprise entre 100 et 1000 mg/ml. La (les) hormone(s) sont alors ajoutées à cette solution en chauffant la solution à une température comprise entre 40 et 90 °C. Le complexe d'inclusion se forme alors. De l'eau purifiée est alors additionnée jusqu'à obtention de la concentration finale en hormone(s) souhaitée, soit de manière extemporanée soit après lyophilisation de la solution précédente.

A cette solution finale est alors éventuellement ajouté du chlorure de sodium pour obtenir une solution physiologique dont le pH peut être ajusté pour mimer le pH nasal.

Dans ce procédé de préparation, la cyclodextrine est dissoute dans de l'eau purifiée à une concentration préférentiellement comprise entre 400 et 500 mg/ml.

La(s) hormone(s) sont alors ajoutées à cette solution de cyclodextrine à une température préférentiellement comprise entre 70 et 90°C.

Ce procédé présente l'avantage essentiel d'obtenir directement une solution aqueuse dans laquelle le complexe est stable et ne se redissocie pas au fil du temps. Ceci est obtenu par ajustement de la concentration de la cyclodextrine dans l'eau et de la température.

En effet, lorsque cette solution est préparée à température ambiante, la cyclodextrine se dissout mais quelle que soit la concentration de la cyclodextrine dans l'eau, l'addition de (des) hormone(s) conduit à une suspension qui ne se solubilise pas même après plusieurs heures d'agitation : il n'y a donc pas formation du complexe.

Lorsque l'on tente de préparer la solution à une température comprise entre 40 et 90 °C, et lorsque la cyclodextrine est dissoute dans l'eau purifiée à une concentration inférieure à 100 mg/ml, le complexe ne se forme pas même après plusieurs heures d'agitation.

Pour des concentrations de cyclodextrine comprises entre 100 mg/ml et 1000 mg/ml, le complexe ne se forme pas ou mal si la température est inférieure à 40 °C.

Les connaissances de l'homme de métier ne permettaient en aucune façon de prévoir ou d'imaginer ce procédé de préparation, objet de la présente invention.

Les concentrations relatives de cyclodextrine et d'hormone(s) sont bien entendu fonction de la nature de la cyclodextrine elle-même et de (des) hormone(s).

Les méthyl-β-cyclodextrines sont les mieux adaptées à la solubilisation d'hormones comme l'estradiol. A titre d'exemple, il faut 10 moles d'hydroxypropyl β-cyclodextrine pour complexer une mole d'estradiol alors que 2 moles de méthyl β-cyclodextrine suffisent pour complexer une mole d'estradiol.

Les cyclodextrines préférées selon l'invention sont les cyclodextrines partiellement méthylées de manière randomisée. La cyclodextrine partiellement méthylée et randomisée est préférentiellement la cyclodextrine dont le degré moyen de substitution par des groupements méthyles est voisin de 1,7.

Un mode de réalisation particulier de l'invention consiste donc à dissoudre une cyclodextrine partiellement méthylée de manière randomisée dans de l'eau purifiée à une concentration proche de 400 mg/ml, de préférence entre 400 et 500 mg/ml, et à ajouter la quantité nécessaire d'estradiol à une température voisine de 80°C.

Le complexe se forme alors et la solution finale est obtenue par addition d'eau purifiée jusqu'à obtention de la concentration en estradiol souhaitée pour la formulation aqueuse nasale.

Dans un mode de réalisation particulier, la concentration finale en estradiol égale environ à 2,14 mg/ml.

Plus particulièrement, la solution contient du chlorure de sodium et son pH a été ajusté à 6.

Les exemples suivants illustrent l'invention mais ne la limitent en aucune façon.

### EXEMPLE 1 :

### Procédé de préparation d'une solution nasale contenant de l'estradiol et une cyclodextrine méthylée partiellement et randomisée (RAMEB)

### (RAMEB / cyclodextrine dont le degré moyen de substitution par des groupements méthyles voisin de 1,7)

La concentration finale en estradiol doit être égale à environ 2,14 mg/ml de solution. Composants pour 201 de solution aqueuse finale :

| | |
|---|---|
| Estradiol hémihydrate | 42,86 g |
| RAMEB | 418,92 g |

### Stade A : Préparation du complexe estradiol / RAMEB

A un litre d'eau purifiée, est additionné le RAMEB et l'ensemble est agité jusqu'à dissolution complète.
L'estradiol est alors ajouté à cette solution et l'ensemble chauffé à 80°C est agité jusqu'à dissolution complète.

### Stade B : Préparation de la solution finale

A la solution obtenue au stade A sont additionnés 18 1 d'eau purifiée. L'ensemble est agité quelques minutes puis 180 g de chlorure de sodium sont ajoutés à la solution précédente. Après dissolution complète du chlorure de sodium, le pH est ajusté à 6 et le volume total de la solution est complète à 201 par addition d'eau purifiée.
A des fins de stérilisation, l'ensemble est filtré sur membrane d'acétate de cellulose de 0,2 µm de porosité.

### EXEMPLE 2 :

### Procédé de préparation d'une solution nasale contenant de l'estradiol, de l'acétate de noréthistérone et une cyclodextrine méthylée partiellement et randomisée (RAMEB)

### (RAMEB / cyclodextrine dont le degré moyen de substitution par des groupements méthyles voisin de 1,7)

La concentration finale en estradiol doit être égale à environ 2,14 mg/ml de solution, la concentration finale en noréthistérone acétate doit être égale à environ 6,39 mg/ml de solution.
Composants pour 201 de solution aqueuse finale :

| | |
|---|---|
| Estradiol hémihydrate | 42,86 g |
| Noréthistérone acétate | 27,8 g |
| RAMEB | 1500 g |

### Stade A : Préparation du complexe estradiol, noréthistérone acétate /RAMEB

A trois litres d'eau purifiée, est additionné le RAMEB et l'ensemble est agité jusqu'à dissolution complète.
L'estradiol et l'acétate de noréthistérone sont alors ajoutés à cette solution et l'ensemble chauffé à 80°C est agité jusqu'à dissolution complète.

### Stade B : Préparation de la solution finale

A la solution obtenue au stade A sont additionnés 16 1 d'eau purifiée. L'ensemble est agité quelques minutes puis 180 g de chlorure de sodium sont ajoutés à la solution précédente.
Après dissolution complète du chlorure de sodium, le pH est ajusté à 6 et le volume total de la solution est complète à 201 par addition d'eau purifiée.
A des fins de stérilisation, l'ensemble est filtré sur membrane d'acétate de cellulose de 0,2 µm de porosité.

### EXEMPLE 3 :

### Procédé de préparation d'une solution nasale contenant de la progestérone et une cyclodextrine méthylée partiellement et randomisée (RAMEB)

### (RAMEB / cyclodextrine dont le degré moyen de substitution par des groupements méthyles voisin de 1,7)

La concentration finale en progestérone doit être égale à environ 8,86 mg/ml de solution. Composants pour 201 de solution aqueuse finale :

| | |
|---|---|
| Progestérone | 177,2 g |
| RAMEB | 1500 g |

### Stade A : Préparation du complexe estradiol / RAMEB

A trois litres d'eau purifiée, est additionné le RAMEB et l'ensemble est agité jusqu'à dissolution complète.
La progestérone est alors ajoutée à cette solution et l'ensemble chauffé à 80°C est agité jusqu'à dissolution complète.

### Stade B : Préparation de la solution finale

A la solution obtenue au stade A sont additionnés 16 litres d'eau purifiée. L'ensemble est agité quelques minutes puis 180 g de chlorure de sodium sont ajoutés à la solution précédente. Après dissolution complète du chlorure de sodium, le pH est ajusté à 6 et le volume total de la solution est complète à 20 litres par addition d'eau purifiée.
A des fins de stérilisation, l'ensemble est filtré sur membrane d'acétate de cellulose de 0,2 µm de porosité.

### EXEMPLE 4 :

### Procédé de préparation d'une solution nasale contenant de la testostérone et une cyclodextrine méthylée partiellement et randomisée (RAMEB)

### (RAMEB / cyclodextrine dont le degré moyen de substitution par des groupements méthyles voisin de 1,7)

La concentration finale en testostérone doit être égale à environ 2,20 mg/ml de solution.

Composants pour 20 de solution aqueuse finale :

| | |
|---|---|
| Testostérone | 43,92 g |
| RAMEB | 418,92 g |

### Stade A : Préparation du complexe estradiol / RAMEB

A un litre d'eau purifiée, est additionné le RAMEB et l'ensemble est agité jusqu'à dissolution complète.
L'estradiol est alors ajouté à cette solution et l'ensemble chauffé à 80°C est agité jusqu'à dissolution complète.

### Stade B : Préparation de la solution finale

A la solution obtenue au stade A sont additionnés 18 litres d'eau purifiée. L'ensemble est agité quelques minutes puis 180 g de chlorure de sodium sont ajoutés à la solution précédente. Après dissolution complète du chlorure de sodium, le pH est ajusté à 6 et le volume total de la solution est complété à 20 litres par addition d'eau purifiée.
A des fins de stérilisation, l'ensemble est filtré sur membrane d'acétate de cellulose de 0,2 µm de porosité.

## Revendications

1. Procédé de préparation d'une solution pour pulvérisation nasale contenant une ou plusieurs hormones sexuelles et une cyclodextrine **caractérisé en ce que** :
- la cyclodextrine est dissoute dans de l'eau purifiée à une concentration comprise entre 100 et 1000 mg/ml,
- la (les) hormones sont ajoutées en chauffant la solution à une température comprise entre 40 et 90 °C,
- de l'eau purifiée est additionnée jusqu'à obtention de la concentration finale en hormone(s) souhaitée, extemporanément ou après lyophilisation de la solution précédente.

2. Procédé selon la revendication 1 **caractérisé en ce que** la cyclodexrine est dissoute dans de l'eau purifiée à une concentration comprise entre 400 et 500 mg/ml et que la (les) hormone(s) sont ajoutées à une température comprise entre 70 et 90°C.

3. Procédé de préparation selon l'une quelconques des revendications 1 ou 2 **caractérisé en ce que** la cyclodextrine est une cyclodextrine méthylée partiellement.

4. Procédé de préparation selon la revendication 3 **caractérisé en ce que** la cyclodextrine est méthylée partiellement et de manière randomisée.

5. Procédé de préparation selon la revendication 4 **caractérisé en ce que** la cyclodexrine méthylée partiellement et de manière randomisée présente un degré moyen de substitution par des groupements méthyles voisin de 1,7.

6. Procédé de préparation selon l'une quelconque des revendications 1, 2, 3, 4 ou 5 **caractérisé en ce que** l'hormone sexuelle utilisée est l'estradiol.

7. Procédé de préparation selon l'une quelconque des revendications 1, 2, 3, 4 ou 5 **caractérisé en ce que** les hormones sexuelles utilisées sont l'estradiol et/ou un progestatif.

8. Procédé de préparation selon la revendication 7 **caractérisé en ce que** le progestatif est un dérivé de la 19-nortéstostérone.

9. Procédé de préparation selon la revendication 8 **caractérisé en ce que** le progestatif est l'acétate de noréthistérone.

10. Procédé de préparation selon l'une quelconque des revendications 1, 2, 3, 4 ou 5 **caractérisé en ce que** l'hormone est la testostérone.

11. Procédé de préparation selon l'une quelconque des revendications 1, 2, 3, 4, 5 ou 6 **caractérisé en ce que** :
- l'on prépare une solution aqueuse de β-cyclodextrine méthylée partiellement et de manière randomisée de concentration comprise entre 400 et 500 mg/ml,
- l'on ajoute sous agitation de l'estradiol et que l'on chauffe cette solution à 80°C,
- que l'on complète le volume par addition d'eau purifiée pour obtenir une concentration finale en estradiol égale environ à 2,14 mg/ml.

12. Procédé de préparation d'une solution aqueuse nasale d'estradiol selon la revendication 11 **caractérisé en ce que** cette solution contient également du chlorure de sodium et que son pH a été ajusté à 6.

## Claims

1. Process for the preparation of a nasal spray solution comprising one or more sex hormones and a cyclodextrin, **characterised in that**:
- the cyclodextrin is dissolved in purified water at a concentration of from 100 to 1000 mg/ml,
- the hormone(s) is/are added while heating the solution at a temperature of from 40 to 90°C,
- purified water is added until the desired final concentration of hormone(s) is obtained, either immediately or after lyophilisation of the above solution.

2. Process according to claim 1, **characterised in that** the cyclodextrin is dissolved in purified water at a concentration of from 400 to 500 mg/ml and the hormone(s) is/are added at a temperature of from 70 to 90°C.

3. Preparation process according to either claim 1 or claim 2, **characterised in that** the cyclodextrin is a partially methylated cyclodextrin.

4. Preparation process according to claim 3, **characterised in that** the cyclodextrin is randomly partially methylated.

5. Preparation process according to claim 4, **characterised in that** the randomly partially methylated cyclodextrin has an average degree of substitution by methyl groups of about 1.7.

6. Preparation process according to any one of claims 1, 2, 3, 4 and 5, **characterised in that** the sex hormone used is oestradiol.

7. Preparation process according to any one of claims 1, 2, 3, 4 and 5, **characterised in that** the sex hormones used are oestradiol and/or a progestogen.

8. Preparation process according to claim 7, **characterised in that** the progestogen is a derivative of 19-nortestosterone.

9. Preparation process according to claim 8, **characterised in that** the progestogen is norethisterone acetate.

10. Preparation process according to any one of claims 1, 2, 3, 4 and 5, **characterised in that** the hormone is testosterone.

11. Preparation process according to any one of claims 1, 2, 3, 4, 5 and 6, **characterised in that**:
- an aqueous solution of randomly partially methylated β-cyclodextrin of a concentration of from 400 to 500 mg/ml is prepared,
- oestradiol is added with stirring and the solution is heated at 80°C,
- the volume is made up by the addition of purified water to obtain a final concentration of oestradiol of about 2.14 mg/ml.

12. Process for the preparation of an aqueous nasal oestradiol solution according to claim 11, **characterised in that** that solution also comprises sodium chloride and that its pH has been adjusted to 6.

## Patentansprüche

1. Verfahren zur Herstellung einer Nasalsprühlösung, die ein oder mehrere Sexualhormone und ein Cyclodextrin enthält, **dadurch gekennzeichnet, daß**:
- das Cyclodextrin in einer Konzentration zwischen 100 und 1000 mg/ml in gereinigtem Wasser gelöst wird,
- das (die) Hormon(e) unter Erhitzen der Lösung auf eine Temperatur zwischen 40 und 90°C zugegeben werden, und
- gereingtes Wasser bis zum Erhalt der angestrebten Hormon-Endkonzentration zusetzt, vor der Anwendung oder nach der Gefriertrocknung der obigen Lösung zugesetzt wird.

2. Verfahren nach Anspruch 1, **dadurch gekennzeichnet, daß** das Cyclodextrin in einer Konzentration zwischen 400 und 500 mg/ml in dem gereinigten Wasser gelöst wird und das (die) Hormon(e) bei einer Temperatur zwischen 70 und 90°C zugegeben werden.

3. Herstellungsverfahren nach einem der Ansprüche 1 oder 2, **dadurch gekennzeichnet, daß** das Cyclodextrin ein teilweise methyliertes Cyclodextrin ist.

4. Herstellungsverfahren nach Anspruch 3, **dadurch gekennzeichnet, daß** das Cyclodextrin ein teilweise und statistisch methyliertes Cyclodextrin ist.

5. Herstellungsverfahren nach Anspruch 4, **dadurch gekennzeichnet, daß** das teilweise und statistisch methylierte Cyclodextrin einen durchschnittlichen Substitutionsgrad durch die Methylgruppen von etwa 1,7 aufweist.

6. Herstellungsverfahren nach einem der Ansprüche 1, 2, 3, 4 oder 5, **dadurch gekennzeichnet, daß** das verwendete Sexualhormon Östradiol ist.

7. Herstellungsverfahren nach einem der Ansprüche 1, 2, 3, 4 oder 5, **dadurch gekennzeichnet, daß** die verwendeten Sexualhormone Östradiol und/oder ein Progestativ ist.

8. Herstellungsverfahren nach Anspruch 7, **dadurch gekennzeichnet, daß** das Progestativ ein 19-Nortestosteron-Derivat ist.

9. Herstellungsverfahren nach Anspruch 8, **dadurch gekennzeichnet, daß** das Progestativ Norethisteron-acetat ist.

10. Herstellungsverfahren nach einem der Ansprüche 1, 2, 3, 4, oder 5, **dadurch gekennzeichnet, daß** das Hormon Testosteron ist.

11. Herstellungsverfahren nach einem der Ansprüche 1, 2, 3, 4, 5 oder 6, **dadurch gekennzeichnet, daß** man:
- eine wäßrige Lösung von teilweise und statistisch methyliertem β-Cyclodextrin in einer Konzentration zwischen 400 und 500 mg/ml herstellt,
- unter Rühren Östradiol zusetzt und diese Lösung auf 80°C erhitzt, und
- das Endvolumen durch Zugabe von gereinigtem Wasser bis zu einer Östradiol-Endkonzentration von ewa 2,14 mg/ml vervollständigt.

12. Verfahren zur Herstellung einer wäßrigen, nasal zu verabreichenden Östradiollösung nach Anspruch 11, **dadurch gekennzeichnet, daß** diese Lösung zusätzlich Natriumchlorid enthält und ihr pH-Wert auf 6 eingestellt wird.
